**Europäisches Patentamt**

**European Patent Office**

**Office européen des brevets**

(11) Publication number: **0 173 710**
**B1**

# EUROPEAN PATENT SPECIFICATION

(12)

(45) Date of publication of patent specification: **02.05.90**

(21) Application number: **85901153.8**

(22) Date of filing: **25.01.85**

(86) International application number:
**PCT/US85/00124**

(87) International publication number:
**WO 85/03879 12.09.85 Gazette 85/20**

(51) Int. Cl.⁵: **A 61 M 1/14**

(54) **PRIMING SYSTEM FOR ULTRAFILTRATION UNIT.**

(30) Priority: **27.02.84 US 583854**

(43) Date of publication of application:
**12.03.86 Bulletin 86/11**

(45) Publication of the grant of the patent:
**02.05.90 Bulletin 90/18**

(84) Designated Contracting States:
**BE DE FR GB SE**

(56) References cited:
**FR-A-2 295 777**
**US-A-3 907 504**
**US-A-3 927 980**
**US-A-3 962 075**
**US-A-4 299 705**

(73) Proprietor: **BAXTER INTERNATIONAL INC. (a**
**Delaware corporation)**
**One Baxter Parkway**
**Deerfield Illinois 60015 (US)**

(72) Inventor: **LEONARD, Ronald, J.**
**19211 Crowley Road**
**Harvard, IL 60033 (US)**

(74) Representative: **MacGregor, Gordon et al**
**ERIC POTTER & CLARKSON 14 Oxford Street**
**Nottingham, NG1 5BP (GB)**

Courier Press, Leamington Spa, England.

## Description

Technical Field

The present invention concerns a novel system for priming an ultrafiltration unit.

Background Art

One of the problems in cardiac bypass surgery is that when the patient's blood has been fully diluted in a bypass circuit with priming solution, addition fluid and cardioplegia solution, the hematocric has dropped to well under normal values. Since the patient cannot take back his own blood volume and the circuit volume, much of this diluted blood and the patient's blood cells and proteins are left in the oxygenator, heat exchanger and tubing. Recently, a high ultrafiltration hemo-dialyzer has been used to concentrate this blood by removal of water so that a reasonable volume of the valuable blood constituents can be given back to the patient. In this operational mode, a dialyzer is used only as an ultrafiltrator so that no dialysis solution flow is required. Ultrafiltration is achieved by drawing a vacuum on the dialysate compartment. Sometimes a blood pump is used, but often a tap is made in the circuit downstream of the bypass circuit aterial pump or venous pump in the oxygenator. Thus the circuit and ultrafiltration unit must be primed without a pump.

Dialyzers may require the rinsing of both the blood compartment and the dialysate compartment to prepare the dialyzer and to guard against the possibility of a hypersensitivity reaction in the patient. Of course the rinsing solution must be discarded.

The present invention is particularly applicable to any type of ultrafiltration unit, including a hemoconcentrator, a dialyzer, a diafilter, etc. Such ultrafiltration units generally include an ultrafiltration membrane which separates a blood compartment from an ultrafiltrate compartment. When a dialyzer is used as an ultrafiltration unit, the dialysate compartment of the dialyzer becomes the ultrafiltrate compartment.

It is an object of the present invention to provide a system for priming an ultrafiltration unit without requiring a pump in the blood line.

Another object of the present invention is to provide a system for priming an ultrafiltration unit with a provision for automatically discarding the priming solution without disconnection or reconnection of the blood set.

A further object of the present invention is to provide a system for priming an ultrafiltration unit, enabling the priming and rinsing of both the blood compartment and the ultrafiltrate compartment, at prescribed flow and volume rates and with an automatic discard of the priming and rinsing solution without disconnection or reconnection of the blood set.

By avoiding the necessity of disconnecting or reconnecting the blood set before, during or after priming and/or rinsing, the sterility compromise concomitant with disconnection or reconnection is obviated.

Other objects and advantages of the present invention will become apparent as the description proceeds.

US—A—4299705 discloses an ultrafiltration system comprising an ultrafiltration unit, for connection to a blood source and having an ultrafiltration membrane which separates a blood compartment from an ultrafiltrate compartment, a blood inlet port, a blood outlet port, and an ultrafiltrate outlet port, and a second inlet port communicating with the ultrafiltrate compartment. The invention is characterised by a feedback tube connecting the blood outlet port to the second inlet port, whereby a low pressure can be applied to the ultrafiltrate outlet port and priming solution introduced at the blood inlet port will be drawn through the blood compartment and then through the feedback tube to the ultrafiltrate compartment and out the ultrafiltrate outlet port, without the requirement of a pump in the blood line.

In the illustrative embodiment, blood inlet tubing is provided for connecting the blood inlet port to a blood source. A first port is provided on the blood inlet tubing for connecting a pressure monitor to the blood inlet tubing. A second port is provided on the blood inlet tubing for connecting a priming solution container to the blood inlet tubing. Blood outlet tubing extends from the blood outlet port. Means are provided for connecting the ultrafiltrate outlet port to a container and means connect the container to a vacuum source.

In the illustrative embodiment, a flow restrictor is interposed in the feedback tube to control the flow rate of the priming solution through the blood path and then into the ultrafiltrate compartment.

The present invention also provides a method for priming an ultrafiltration unit connected to a blood source without requiring a pump in the blood line, as defined in Claim 8.

A more detailed explanation of the invention is provided in the following description and claims, and is illustrated in the accompanying drawings.

Brief Description of the Drawings

Figure 1 is a schematic diagram of a system for priming an ultrafiltration unit, constructed in accordance with the principles of the present invention.

Figure 2 is a schematic diagram of the system of Figure 1, after a first step of priming has been accomplished.

Figure 3 is a schematic diagram of the system of Figure 1, after a second step of priming has been accomplished.

Figure 4 is a schematic diagram of the system of Figure 1, after the system has been fully primed.

Detailed Description of the Illustrative Embodiment

Referring to the Figures, an ultrafiltration unit 10, in the exemplary form of a dialyzer, is illustrated having a blood inlet port 12, a blood outlet port 14, a second inlet port 16 and an ultrafiltrate outlet port 18. In a dialyzer, ports 16 and 18 would be the dialysate inlet port and the dialysate outlet port, respectively. Ultrafiltration unit 10 includes a suitable ultrafiltration membrane separating the blood compartment from the ultrafiltrate compartment, as is well-known in the art.

A blood inlet port 20 connects blood inlet port 12 to the outlet 22 of an oxygenator 24. A blood outlet tube 26 connects the blood outlet port 14 to the inlet 28 of oxygenator 24. This connection could be directly to the oxygenator illustrated or through a cardiotomy reservoir. Additionally, blood might be introduced into a blood bag or other container directly from the hemoconcentrator. The illustrated method is for explanation only and not to restrict the choice of blood inlet source or outlet final destination.

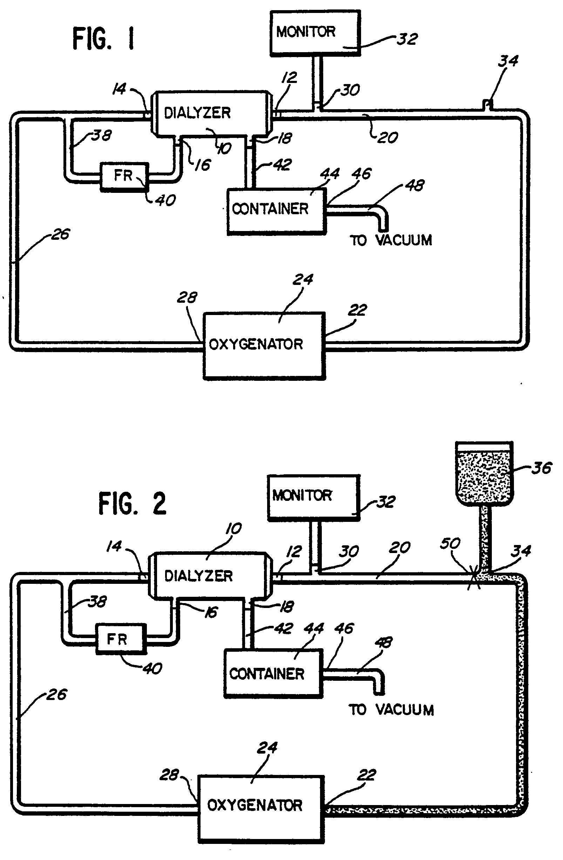

Blood inlet tube 20 includes a first port 30 for enabling the connection of a pressure monitor 32 to the blood inlet tube 20. The blood inlet tube 20 also has a second port 34 for enabling the connection of a priming solution container 36 (Figures 2—4) or rinsing solution container to the blood inlet tube 20.

A feedback tube 38 having a flow restrictor 40 is connected from blood outlet port 14 to the second inlet port 16. The second inlet port 16 communicates with the ultrafiltrate compartment of unit 10. The flow restrictor 40 operates to control the flow rate through the ultrafiltration unit 10 as will be explained below.

A priming solution output tube 42 connects ultrafiltrate outlet port 18 to a container 44 with the outlet 46 of container 44 being coupled to a vacuum source via tubing 48. Often the hospital has a wall vacuum which is connected to tubing 48.

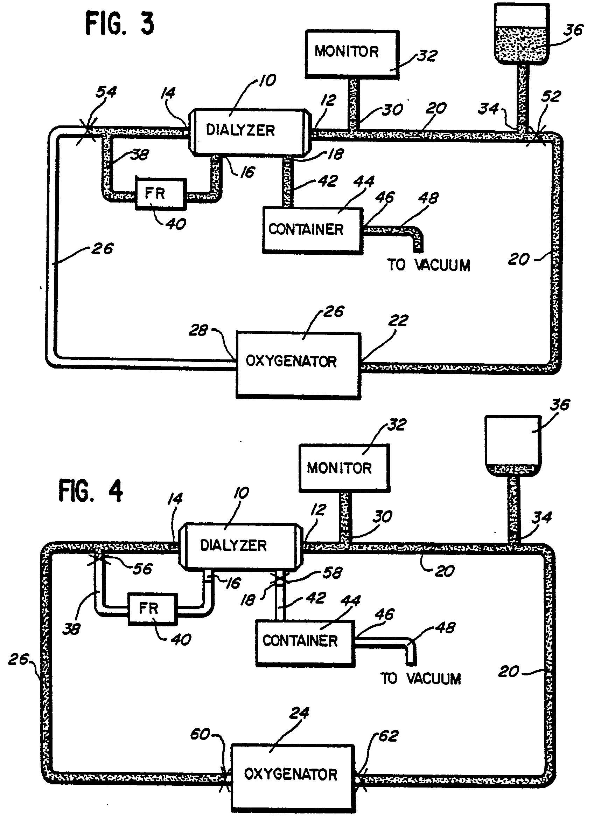

The operation of the system will now be explained. Referring to Figure 2, blood inlet tubing 20 is clamped at point 50 and a container 36 of priming solution such as saline solution, is attached to port 34. The portion of tubing 20 between point 50 and the oxygenator is then primed. Referring to Figure 3, the clamp is removed at point 50, the blood inlet tubing is clamped at point 52 (upstream of port 34) and the blood outlet tubing 26 is clamped at point 54 (downstream of the feedback tube 38). A vacuum of determined level such as 500 mm mercury is applied at ultrafiltrate outlet port 18. The priming fluid will be drawn from container 36 through the blood compartment of ultrafiltration unit 10, to point 54, through flow restrictor 40, into the ultrafiltrate compartment via second inlet port 16, out of ultrafiltrate outlet port 18 and to drain via tube 48. It can be seen that flow restrictor 40 controls the flow rate of the fluid into the ultrafiltrate compartment.

As the priming fluid flows through the blood compartment of ultrafiltration unit 10, some of it will be ultrafiltered through the membrane. The ultrafiltration unit will be rinsed, flushed and primed with the solution automatically discarded to drain. After about five minutes, in the illustrative embodiment, approximately 800 to 900 ml of priming solution will have passed through the ultrafiltration unit 10.

Referring to Figure 4, after the ultrafiltration unit 10 has been primed, the clamps at points 52 and 54 are removed and clamps are provided at points 56 and 58, to stop the feedback line and to remove the vacuum. In this manner, the remaining portion of the blood outlet tubing 26 is primed using the remaining solution in container 36 and the line is clamped at points 60 and 62. The system is now ready for use. Clamp 56 remains in place but clamps 58, 60 and 62 are removed and the correct negative pressure is applied at ultrafiltrate outlet port 18 to control ultrafiltration.

It can be seen that a novel system has been disclosed for priming and rinsing an ultrafiltration unit, such as a dialyzer, hemoconcentrator or diafilter, without requiring the use of a pump in the blood line. Both the blood compartment and ultrafiltrate compartment are rinsed at a controlled rate and the solution is automatically discarded without disconnection or reconnection of the blood tubing which could result in sterility compromise.

## Claims

1. An ultrafiltration system comprising an ultrafiltration unit (10) for connection to a blood source and having an ultrafiltration membrane which separates a blood compartment from an ultrafiltrate compartment, a blood inlet port (12), a blood outlet port (14), and an ultrafiltrate outlet port (18), and a second inlet port (16) communicating with the ultrafiltrate compartment, characterised by a feedback tube (38) connecting the blood outlet port (14) to the second inlet port (16) whereby a low pressure can be applied to the ultrafiltrate outlet port (18) and priming solution introduced at the blood inlet port will be drawn through the blood compartment and then through the feedback tube to the ultrafiltrate compartment and out the ultrafiltrate outlet port, without the requirement of a pump in the bloodline.

2. An ultrafiltration system according to Claim 1, including blood inlet tubing (20) for connecting the blood inlet port (12) to a blood source and a port (34) on the blood inlet tubing for connecting a priming solution container (36) to the blood inlet tubing.

3. An ultrafiltration system according to Claim 1 or 2, including means (42) for connecting the ultrafiltrate outlet port (18) to a container (44) and means (46, 48) for connecting the container to a vacuum source.

4. An ultrafiltration system according to Claim 1, 2 or 3, including a flow restrictor interposed in the feedback tube to control the flow rate into the ultrafiltrate compartment.

5. An ultrafiltration system according to Claim 1, 2, 3 or 4, comprising means (42, 48) for connecting the ultrafiltration outlet port (18) to a vacuum source.

6. An ultrafiltration system according to any preceding claim, in which said ultrafiltration unit is a hemoconcentrator.

7. An ultrafiltration system according to any one of Claims 1 to 6, in which said ultrafiltration unit is a dialyzer (10), said second inlet port (16) is a dialysis solution inlet port and said ultrafiltrate outlet port is a dialysis solution outlet port (18).

8. A method for priming an ultrafiltration unit connected to a blood source without requiring a pump in the blood line, the unit comprising a feedback tube (38) connected between a blood outlet port (14) and a second inlet port (16) of the ultrafiltration unit (10), and an ultrafiltration membrane which separates a blood compartment from an ultrafiltrate compartment and a blood inlet port (12), said blood outlet port (14) and said second inlet port (16) communicating with the ultrafiltrate compartment, and an ultrafiltrate outlet port (18), the method comprising the steps of introducing priming solution to the blood inlet port (12) and applying a low pressure to the ultrafiltrate outlet port (18) to draw the priming solution through the blood compartment, through the ultrafiltrate compartment and out of the ultrafiltrate outlet port.

9. The method according to Claim 8, including the sequential steps of connecting a priming solution container (36) to blood inlet tubing (20) connected to the blood inlet port (12), clamping the tubing (20) upstream of the blood inlet port (12), introducing priming solution into the tubing upstream of the clamp, clamping tubing (26) connected to the blood outlet port (14) at a position downstream of the feedback tube (38); and unclamping the tubing (20) upstream of the blood inlet port (12) so as to introduce priming solution to the blood inlet port.

**Patentansprüche**

1. Ultrafiltrationssystem mit einer Ultrafiltriereinheit (10) zum Anschluß an eine Blutversorgungsquelle und mit einer Ultrafiltrationsmembran, die eine Blutkammer von einer Ultrafiltratkammer trennt, bei welchem auch eine Bluteinströmöffnung (12), eine Blutausströmöffnung (14) und eine Auströmöffnung (18) für das Ultrafiltrat, sowie eine zweite Einströmöffnung (16) vorgesehen sind, welche mit der Ultrafiltratkammer in Verbindung steht, gekennzeichnet durch eine Rückführschlauchleitung (38), die die Bluteinströmöffnung (14) mit der zweiten Einströmöffnung (16) verbindet, wobei an die Ausströmöffnung (18) für das Ultrafiltrat ein niedriger Druck anlegbar ist und eine über die Bluteinströmöffnung eingebrachte Anpumplösung durch die Blutkammer gesaugt und anschließend durch die Rückführschlauchleitung zur Ultrafiltratkammer gesaugt und aus der Ausströmöffnung für das Ultrafiltrat ausgetragen wird, ohne daß eine Pumpe in der Blutströmungsleitung erforderlich ist.

2. Ultrafiltrationssystem nach Anspruch 1, mit einer Schlauchleitung (20) für den Blutzustrom, welche die Bluteinströmöffnung (12) mit einer Blutversorgungsquelle verbindet, und mit einer Verbindungsöffnung (34) auf der Schlauchleitung für den Blutzustrom, welche einen Behälter (36) mit Anpumplösung mit der Schlauchleitung für den Blutzustrom verbindet.

3. Ultrafiltrationssystem nach Anspruch 1 oder 2, mit einer Einrichtung (42) zum Verbinden der Ausströmöffnung (18) für das Ultrafiltrat mit einem Behälter (44), sowie mit einer Einrichtung (46, 48) zum Anschluß des Behälters an eine Unterdruckquelle.

4. Ultrafiltrationssystem nach Anspruch 1, 2 oder 3, mit einem in der Rückführschlauchleitung zwischengeschalteten Durchflußbegrenzer zur Beeinflussung der Durchflußrate in die Ultrafiltratkammer.

5. Ultrafiltrationssystem nach Anspruch 1, 2, 3 oder 4, welches eine Einrichtung (42, 48) zum Verbinden der Ausströmöffnung (18) für das Ultrafiltrat mit einer Unterdruckquelle aufweist.

6. Ultrafiltrationssystem nach einem der vorhergehenden Ansprüche, bei welchem die Ultrafiltriereinheit eine Hämokonzentrationsvorrichtung ist.

7. Ultrafiltrationssystem nach einem der Ansprüche 1 bis 6, bei welchem die Ultrafiltriereinheit ein Dialysegerät (10) ist, die zweite Einströmöffnung (16) eine Zulauföffnung für eine Dialyselösung ist, und die Ausströmöffnung für das Ultrafiltrat eine Öffnung (18) für den Ablauf der Dialyselösung ist.

8. Verfahren zum Anpumpen einer an eine Blutversorgungsquelle angeschlossenen Ultrafiltriereinheit, ohne daß eine Pumpe in der Blutströmsleitung erforderlich ist, wobei die Einheit eine Rückführschlauchleitung (38), welche zwischen eine Blutausströmöffnung (14) und eine zweite Einströmöffnung (16) der Ultrafiltriereinheit geschaltet ist, sowie eine Ultrafiltrationsmembran, die eine Blutkammer von einer Ultrafiltratkammer trennt, und eine Bluteinströmöffnung (12) aufweist, wobei die Blutausströmöffnung (14) und die zweite Einströmöffnung (16) mit der Ultrafiltratkammer und einer Ausströmöffnung (18) für das Ultrafiltrat in Verbindung stehen, wobei das Verfahren die Verfahrensschritte des Einleitens einer Anpumplösung zur Bluteinströmöffnung (12) und des Anlegens eines niedrigen Drucks an die Ausströmöffnung (18) für das Ultrafiltrat aufweist, um die Anpumplösung durch die Blutkammer, durch die Ultrafiltratkammer und aus der Ausströmöffnung für das Ultrafiltrat zu saugen.

9. Verfahren nach Anspruch 8, welches die aufeinanderfolgenden Schritte des Anschließens eines Behälters (36) mit Anpumplösung an die Schlauchleitung (20) für den Blutzustrom aufweist, welche mit der Bluteinströmöffnung (12) verbunden ist, sowie des Abklemmens der Schlauchleitung (20) strömungsaufwärts von der Bluteinströmöffnung (12), des Einleitens einer Anpumplösung in die Schlauchleitung oberhalb der Schlauchklemmung; des Abklemmens der mit der Blutausströmöffnung (14) verbundenen Schlauchleitung (26) an einer abströmseitig von der Rückfuhrschlauchleitung (38) befindlichen Stelle; und des Lösens der Schlauchklemme an der Schlauchleitung (20) oberhalb der Bluteinströmöffnung (12), um die Anpumplösung in die Bluteinströmöffnung fließen zu lassen.

**Revendications**

1. Système d'ultrafiltration comprenant une unité d'ultrafiltration (10) pour raccordement à une source de sang et comportant une membrane d'ultrafiltration qui sépare un compartiment de sang d'un compartiment d'ultrafiltrat, un orifice d'entrée de sang (12), un orifice de sortie de sang (14) et un orifice de sortie d'ultrafiltrat (18), et un deuxième orifice d'entrée (16) qui communique avec le compartiment d'ultrafiltrat, caractérisé par un tube de retour (38) reliant l'orifice de sortie de sang (14) au deuxième orifice d'entrée (16) de sorte qu'une basse pression peut être appliquée à l'orifice de sortie d'ultrafiltrat (18) et que la solution d'amorçage introduite à l'orifice d'entrée de sang est aspirée à travers le compartiment de sang puis à travers le tube de retour jusqu'au compartiment d'ultrafiltrat et elle sort à l'orifice de sortie d'ultrafiltrat, sans nécessiter de pompe dans la ligne de sang.

2. Système d'ultrafiltration suivant la revendication 1, comprenant un tuyau d'entrée de sang (20), pour relier l'orifice d'entrée de sang (12) à une source de sang, et un orifice (34) prévu sur le tuyau d'entrée de sang pour relier un récipient de solution d'amorçage (36) au tuyau d'entrée de sang.

3. Système d'ultrafiltration suivant la revendication 1 ou 2, comprenant des moyens (42), pour relier l'orifice de sortie d'ultrafiltrat (18) à un récipient (44), et des moyens (46, 48) pour relier le récipient à une source de vide.

4. Système d'ultrafiltration suivant la revendication 1, 2 ou 3, comprenant un limiteur de débit interposé dans le tube de retour pour régler le débit introduit dans le compartiment d'ultrafiltrat.

5. Système d'ultrafiltration suivant la revendication 1, 2, 3 ou 4, comprenant des moyens (42, 48) pour relier l'orifice de sortie d'ultrafiltrat (18) à une source de vide.

6. Système d'ultrafiltration suivant l'une quelconque des revendications précédentes, dans lequel ladite unité d'ultrafiltration est un hémoconcentrateur.

7. Système d'ultrafiltration suivant l'une quelconque des revendications 1 à 6, dans lequel ladite unité d'ultrafiltration est un dialyseur (10), ledit deuxième orifice d'entrée (16) est un orifice d'entrée de solution de dialyse et ledit orifice de sortie d'ultrafiltrat est un orifice de sortie de solution de dialyse (18).

8. Procédé pour l'amorçage d'une unité d'ultrafiltration raccordée à une source de sang, sans necessiter de pompe dans la ligne de sang, l'unité comprenant un tube de retour (38) connecté entre un orifice de sortie de sang (14) et un deuxième orifice d'entrée (16) de l'unité d'ultrafiltration (10), et une membrane d'ultrafiltration qui sépare un compartiment de sang d'un compartiment d'ultrafiltrat et un orifice d'entrée de sang (12), ledit orifice de sortie de sang (14) et ledit deuxième orifice d'entrée (16) communiquant avec le compartiment d'ultrafiltrat, et un orifice de sortie d'ultrafiltrat (18), le procédé comprenant les opérations d'introduction d'une solution d'amorçage à l'orifice d'entrée de sang (12) et l'application d'une basse pression à l'orifice de sortie d'ultrafiltrat (18) pour déplacer la solution d'amorçage à travers le compartiment de sang et à travers le compartiment d'ultrafiltrat et pour l'évacuer par l'orifice de sortie d'ultrafiltrat.

9. Procédé suivant la revendication 8, comprenant les opérations successives de connexion d'un récipient de solution d'amorçage (36) au tuyau d'entrée de sang (20) raccordé à l'orifice d'entrée de sang (12), de pincement du tuyau (20) en amont de l'orifice d'entrée de sang (12), d'introduction de la solution d'amorçage dans le tuyau en amont de la pince, de pincement du tuyau (26) raccordé à l'orifice de sortie de sang (14) à une position en aval du tube de retour (38), et de libération du tuyau (20) en amont de l'orifice d'entrée de sang (12) de façon à introduire la solution d'amorçage à l'orifice d'entrée de sang.

FIG. 1

FIG. 2

FIG. 3

MONITOR  *32*

*36*

*54*  *14*  *10*  *12*  *30*  *20*  *34*  *52*

DIALYZER

*38*  *16*  *18*

FR  *42*  *44*  *46*  *48*

*40*

CONTAINER

TO VACUUM  *20*

*26*

*28*  *26*  *22*

OXYGENATOR

*32*

MONITOR  *36*

FIG. 4

*14*  *10*  *12*  *30*

DIALYZER

*56*  *16*  *58*  *20*

*38*  *18*  *42*  *44*  *46*

FR

*40*  CONTAINER  *48*

*26*  TO VACUUM  *20*

*60*  *24*  *62*

OXYGENATOR